(19) Europäisches Patentamt
European Patent Office
Office européen des brevets

(11) Veröffentlichungsnummer: **0 100 413**
**B1**

(12) # EUROPÄISCHE PATENTSCHRIFT

(45) Veröffentlichungstag der Patentschrift: **05.02.86**

(51) Int. Cl.⁴: **C 12 Q 1/52**, G 01 N 33/52

(21) Anmeldenummer: **83105503.3**

(22) Anmeldetag: **03.06.83**

(54) **Verfahren und analytische Mittel zur Aktivitätsbestimmung von Glutamat-Oxalacetat-Transaminase.**

(30) Priorität: **09.06.82 DE 3221730**

(43) Veröffentlichungstag der Anmeldung:
**15.02.84 Patentblatt 84/7**

(45) Bekanntmachung des Hinweises auf die Patenterteilung:
**05.02.86 Patentblatt 86/6**

(84) Benannte Vertragsstaaten:
**AT BE CH DE FR GB IT LI LU NL SE**

(56) Entgegenhaltungen:
**DE - A - 2 834 706**
**DE - A - 2 911 481**
**DE - A - 3 026 854**
**US - A - 3 875 014**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH, Sandhofer Strasse 116, D-6800 Mannheim 31 (DE)**

(72) Erfinder: **Deneke, Ulfert, Dr. rer.nat., Birkenweg 5, D-6149 Rimbach-Zotzenbach (DE)**
Erfinder: **Gruber, Wolfgang, Dr.phil.nat., Am Oberanger 6, D-8132 Tutzing-Unterzeismering (DE)**
Erfinder: **Batz, Hans-Georg, Dr.rer.nat., Traubingerstrasse 63, D-8132 Tutzing (DE)**

**EP 0 100 413 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren und analytische Mittel zur Aktivitätsbestimmung des Enzyms Glutamat-Oxalacetat-Transaminase (GOT), welches auch auf Reagenzträgern, wie Teststreifen, durchgeführt werden kann. Das Verfahren eignet sich zur Bestimmung der GOT in biologischen Flüssigkeiten, wie Serum, Urin oder in anderen Materialien.

Die Bestimmung der genannten Transaminase in Plasma, Serum, Geweben und anderem biologischem Material ist von großer diagnostischer Bedeutung bei der Diagnose und Differenzialdiagnose, z.B. von Leber-, Herz- und Muskelverkrankungen. (Zur Bedeutung dieser Bestimmungen vergleiche z.B. H.U. Bergmeyer, Methoden der enzymatischen Analyse, 3. Auflage, 1974, Verlag Chemie Weinheim, Band I, Seite 6 - 74). Es sind daher verschiedene Verfahren zur Bestimmung der GOT beschrieben worden, die jedoch alle mit Nachteilen behaftet sind. (Vergleiche Bergmeyer loc cit. S. 769 - 799).

Die GOT katalysiert folgende Reaktionen:

1a) Aspartat $+\alpha$-Ketoglutarat ($\alpha$-KG) $\xrightleftharpoons{GOT}$
Oxalacetat
(O.AA) + Glutamat

b) Alaninsulfinsäure $+\alpha$-KG $\xrightarrow{GOT}$
Pyruvat + $SO_2$ + Glutamat

Zur Bestimmung der Aktivität dieses Enzyms sind nun verschiedene Verfahren bekannt geworden, die entweder die Abnahme eines der Ausgangsprodukte oder die Geschwindigkeit mit der eines der Endprodukte der Reaktionsgleichungen 1a oder 1b gebildet wird, messen.

So beschreibt A. Karmen (J. Clin. Invest. 34, 133 - 135 (1955) einen Test, bei dem das in Gleichung 1a) gebildete OAA wie folgt gemessen wird:

2) OAA + NADH $+H^{\oplus}$ $\xrightarrow{\text{Malat-Dehydrogenase}}$
Malat + NAD⊕

Die Abnahme der Absorption von NADH pro Minute, gemessen bei 365 nm, ist Meßsignal.

Ein anderes Verfahren zum Nachweis der GOT beschreiben S. Reitmann und S. Fränkel in Am. J. Clin. path. 28, (1957), 56 - 63. Hierbei wird das durch GOT nach Gleichung 1a) gebildete OAA gleichzeitig mit dem im Test vorhandenen $\alpha$-KG mit Dinitrophenylhydrazin zu dem entsprechenden farbigen Hydrazon umgesetzt, welches nach Alkalisierung bei 550 nm bestimmt werden kann. Die Berechnung ist kompliziert aber möglich, weil das Hydrazon von$\alpha$ -Ketoglutarat einerseits und das Hydrazon von OAA andererseits im Meßbereich unterschiedliche Extinktionen aufweisen.

Man kann das nach Gleichung 1a) gebildete OAA aber auch mit Diazoniumsalzen zu einem Farbstoff kuppeln, der dann photometrisch ausgewertet werden kann. Ein solches Verfahren beschreibt A.L. Babson (Clin. Chem. 6 (1960), 394). Hiermit läßt sich die GOT-Aktivität messen. Die Menge des gebildeten Azofarbstoffes kann im sichtbaren Licht gemessen werden. Weiter ist in der DE-A 29 11 481 ein Nachweis der GOT über OAA, ausgehend von Gleichung 1a) in folgender Reaktionskette beschrieben:

3a) OAA $\xrightarrow{\text{OAA-Decarboxilase}}$
Pyruvat + $CO_2$

b) Pyruvat + Phosphat + $O_2$ $\xrightarrow{\text{Py-OD}^{*}}$
Acetyl-Phosphat +
$CO_2$ + $H_2O_2$

c) $2H_2O_2$ + Dimethylanilin + 4-Aminoantipyrin $\xrightarrow{\text{POD}^{**}}$
Chinondiiminfarbstoff + $2H_2O$

Die Extinktionszunahme des gebildeten Farbstoffes wird bei 525 bis 600 nm gemessen.

In der DE-A28 34 706 ist ein GOT-Farbtest beschrieben, der das in der Rückreaktion von Gleichung la) gebildete $\alpha$-KG in folgender Reaktionskette mißt:

4a) $\alpha$- KG $+\mu$ -Aminobutyrat $\xrightarrow{\text{Glutamat- -Aminobutyrat-Transaminase}}$
Succinatsemialdehyd + Glutamat

b) Succinatsemialdehyd + NADP + $\xrightarrow{\text{Succinatsemialdehyd-Dehydrogenase}}$
Sucinat + NADPH + H⊕

c) NADPH + Tetrazoliumsalz $\xrightarrow{\text{Diaphorase}}$
Formazan + NADP⊕

Das gebildete Formazan wird bei geeigneter Wellenlänge gemessen.

* PyOD = Pyruvatoxidase

** POD = Peroxidase

U. Lippi und G. Guidi (Klin. chem. Akta 28 (1970), 431 - 437) maßen die GOT mit Hilfe des in Gleichung 1a) freigesetzten Glutamats, in dem sie folgende 2 Indikatorreaktionen nachschalteten:

5a) Glutamat + $\xrightarrow{\text{Glutamat-Dehydrogenase}}$
$\alpha$-KG+
$NH_3$ + N.ADH + H⊕

b) NADH + Tetrazoliumsalz $\xrightarrow{\text{Phenazinmethosulfat}}$
Formazan + NAD⊕

R.B. Domecq, M. Carta und E.F. de Armorky (Biochem. Klinika 2, (1971), 25 - 36) kombinierten zur Bestimmung der GOT die Reaktionsfolge von Gleichung la), 2) und 5b). Dadurch wird die Abnähme der NADH-Menge,

gemessen als Formazan-Farbstoff, im jeweiligen Test zur Meßgröße. Sie kann im sichtbaren Licht verfolgt werden und ist ebenfalls ein Maß für die Aktivität an GOT in der Probe.

Für die Messung des nach Ib) aus Alaninsulfinsäure ge- bildeten Pyruvat ist z.B. in der DE-A 3o 26 854 der Nachweis mit NADH und LDH entsprechend folgender Gleichung beschrieben:

6) Pyruvat + NADH + H⊕ $\xrightarrow{\text{Lactatdehydrogenase}}$ Lactat
+ NAD⊕

Die Abnahme des NADH, gemessen bei 365 nm, ist Meßsignal.

Alle hier beschriebenen Testverfahren zur Bestimmung der GOT-Aktivität weisen verschiedene gravierende Mängel auf, die ihre Anwendung verteuern oder erschweren. So erlauben die Verfahren nach A. Karmen bzw. nach Serono DE-A 3o 26 8541 zwar die Messung der Extinktionsänderung pro Minute von NADH, was vorteilhaft ist, es muß aber ein UV-Test angewandt werden. Wie dem Fachmann bekannt ist, sind optische Meßeinrichtungen zur Messung im UV-Licht aber besonders aufwendig und daher teurer als Meßanordnungen, die Absorptionsänderungen im sichtbaren Licht messen können. Ein weiterer schwerwiegender Mangel ist die Tatsache, daß eine Abnahme der NADH-Extinktion gemessen werden muß. In einem solchen Test kann aus meßtechnischen Gründen nur eine begrenzte Konzentration an NADH vorhanden sein. Das hat zur Folge, daß die hier verwendeten Indikatorenzyme MDH bzw. LDH nicht mit ihrem Substrat NADH gesättigt sind und demnach nicht maximale Reaktionsgeschwindigkeit erreichen. Das muß durch erhöhten Enzymeinsatz ausgeglichen werden. Dies ist nicht nur unwirtschaftlich, sondern beinhaltet auch die Gefahr, daß andere, störende Enzymaktivitäten in größerem Maße in das Testsystem eingeschleppt werden. Ein weiterer Nachteil der niedrigen NADH-Konzentration ergibt sich daraus, daß sowohl an Analysenautomaten als auch bei manuellem Test zwischen Zugabe der Probe und Beginn der Messung eine gewisse Zeit vergeht. Ist nun eine große Aktivität der GOT in der Probe vorhanden, so wird bereits ein großer Teil des NADH vor Beginn der Messung verbraucht. Deshalb mißt man gerade bei stark erhöhten GOT-Werten, die Hinweise auf ein pathologisches Geschehen im Körper liefern, zu niedrige oder keine Aktivität. Dies ist ein schwerer Mangel der Bestimmungen.

Bei der Methode nach Reitmann und Fränkel werden die gebildeten Hydrazone im sichtbaren Licht bestimmt. Das ermöglicht die Anwendung einfacher und damit preisgünstiger photometrischer Einrichtungen. Dafür ist die Methode sehr unempfindlich und benötigt die sehr lange Inkubationszeit von 1 Stunde. Erst dann hat sich genügend OAA gebildet, so daß die Hydrazonbildung eingeleitet werden kann. Diese benötigt eine weitere Inkubationszeit. Außerdem wird daß im Testansatz erforderliche α-KG ebenfalls zu einem gefärbten Hydrazon umgesetzt. Dadurch ergeben sich für die Auswertung dieses Tests äußerst komplizierte Verhältnisse, da gleichzeitig die Abnahme von α-KG-Hydrazon und die Zunahme von OAA-Hydrazon berücksichtigt werden müssen. Dem Fachmann ist daher schon seit langem bekannt, daß bei dieser Methode Fehler von 20 bis 3o% hingenommen werden müssen. Grundsätzlich ähnliche Probleme ergeben sich bei der Methode nach Babson. Zwar kann auch hier im sichtbaren Licht gemessen werden und weil α-KG mit Diazoniumsalz nicht interferiert, ist die Auswertung wesentlich einfacher, aber auch hier muß zunächst OAA durch längere Inkubation erzeugt werden. Anschließend muß die Farbbildung in einem weiteren Inkubationsschritt durchgeführt werden. Weiter ist dem Fachmann bekannt, daß viele in den zu untersuchenden Lösungen enthaltene Substanzen ebenfalls mit dem Diazoniumsalz zu Farbstoffen kuppeln, die die Bestimmung stören, weil sie zu hohe Werte vortäuschen. Diese Fehler müssen erst durch einen Leerwert eliminiert werden. So enthält z.B. Serum immer wechselnde Menge Acetessigsäure, die Oxalacetat, das sich erst in der GOT-Reaktion bilden soll, vortäuscht.

Bei dem Verfahren nach DE-A 29 11 481 erweist sich als Nachteil, daß insgesamt 4 verschiedene enzymatische Reaktionen gekoppelt werden müssen. Das führt in jedem Fall zu einer längeren lag-phase, bis in der gesamten Kette alle Enzyme Substratsättigung erreicht haben. Weiter ist die Gefahr, störende Fremdund Nebenaktivitäten in den Test einzuschleppen, um so größer, je mehr Enzyme an der Reaktionskette beteiligt sind. Außerdem steigen mit jedem Enzym die Kosten pro Test. Dagegen ist die Möglichkeit dieses Testes, kinetisch eine Farbstoffzunahme zu verfolgen, günstig.

Die gleichen Nachteile des vielfach gekoppelten Testes zeigen sich auch bei dem GOT-Test gemäßDE-A 28 34 7o6. wobei hier noch die Messung mit OAA als Substrat dazukommt. OAA als β-Ketocarbonsäure ist recht instabil und neigt zur Decarboxilierung.

Die Methode nach Lippi und Guidi mißt ebenfalls im sichtbaren Bereich. Sie benötigt nur noch einen Inkubationsschritt, der allerdings mit einer Dauer von 55 Minuten ebenfalls sehr lang ist. Auch muß die Reaktion dann mit der unangenehm zu handhabenden Salzsäure gestoppt werden. Ein weiterer Nachteil ist, daß im Serum relativ große Mengen an Glutaminsäure enthalten sind. Sie täuschen gemäß Gleichung 5a) im Test GOT vor. Deshalb muß auch hier ein Leerwert mitgeführt werden.

Das Verfahren von Domecq et al benötigt wieder 2 In- kubationsschritte. Da es letztlich die Abnahme von NADH, entsprechend der Methode von Karmen mißt, gelten hier die gleichen Nachteile, wie dort aufgezeigt. Sie treten noch stärker hervor, weil aus meßtechnischen Gründen noch weniger NADH eingesetzt werden kann.

Vorteilhaft ist die Messung im sichtbaren Licht. Auch kann hier der völlige Verbrauch von NADH durch zu hohe Aktivität von GOT die Messung verfälschen. Insgesamt aber ist, wie dem Fachmann ohne weiteres ersichtlich, das Verfahren sehr aufwendig und störanfällig, da es höchste Ansprüche an die präzise Dosierung von NADH in jedem Meßwert stellt.

Aufgabe der vorliegenden Erfindung ist es also, einen GOT-Farbtest zu schaffen, der mit einer möglichst kurzen Reaktionskette und damit kurzen Reaktionszeit auskommt, die Messung des Reaktionsproduktes im sichtbaren Licht erlaubt und nicht durch Nebenreaktionen der Enzyme oder Substrate gestört wird.

Versuche die in Gleichung Ib beschriebene Reaktion mit den in Gleichungen 3b und 3c beschriebenen Reaktionen zu koppeln und so eine enzymatische Reaktion (3a) zu vermeiden und ein in sichtbarem Licht meßbares Reaktionsprodukt zu erhalten, schlugen fehl. Man findet keinerlei Farbbildung, auch bei höchstens GOT-Aktivitäten. Auch bei Ersatz des Dimethylanilins durch andere Aniline oder Phenole, die als Kupplungskomponenten für die sogenannten PAP-Systeme bekannt geworden sind, erhält man keine Reaktion, ebenfalls dann nicht, wenn man 4-Aminoantipyrin durch andere kupplungsfähige, nucleophile Reagenzien wie 3-Methyl-benzthiazolonhydrazon-(2) oder dessen 6-Sulfonylverbindung ersetzt. Aber auch die gesamte Stoffklasse der heteroaromatischen Azine, die mit $POD/H_20_2$ intensiv gefärbte Radikale. liefern oder o-Toluidin oder Tetramethylbenzidin, die ebenfalls tiefgefärbte Radikale bilden, geben im Test keine Reaktion. Als Ursache konnte nachgewiesen werden, daß sowohl Alaninsulfinsäure, welche als Substrat der GOT dient und daher in großen Mengen im Test eingesetzt werden muß, als auch das in der GOT-Reaktion gebildete Sulfit durch ihre starke Reduktionswirkung und durch ihre Fähigkeit, selbst oxidative Kupplungen einzugehen, die Farbbildung unterdrücken. Überraschend konnten jedoch Substrate gefunden werden, mit denen die Gleichung 3c weder durch $SO_2$ noch durch Alaninsulfinsäure gestört wird. Solche Verbindungen sind z.B. N-(3'-sulfonyl-4'-hydroxy-5'-chlorphenyl)-4-aminoantipyrin (5HYC-AA) und 4,5-Bis-[4-dimethyl-aminophenyl]-2-[3,5-dimethoxy-4-hydroxyphenyl]-imidazol (B-DDH-Imidazol).

Erfindungsgemäß wird deshalb ein Verfahren zur Aktivitäts- bestimmung von Glutamat-Oxalacetat-Transaminase (GOT) vorgeschlagen, bei dem Alaninsulfinsäure mit α-Ketoglutarat unter Katalyse von GOT zu Pyruvat, $SO_2$ und Glutamat umgesetzt wird, das Pyruvat mit Phosphat und Sauerstoff unter Katalyse mit Pyruvatoxidase zu Acetylphosphat, $CO_2$ und $H_20_2$ reagiert und das $H_20_2$ mittels einer Peroxidase katalysierten Oxidation eines Chromogens bestimmt wird, dadurch gekennzeichnet, daß als Chromogen N-(3'-Sulfonyl-4'-hydroxy-5'-chlorphenyl)-4aminoantipyrin (SHYC-AA) und 4,5-Bis-/4-dimethyl-aminophenyl[-2-]3,5-dimethoxy-4-hydroxyphenyl]-imidazol (B-DDH-Imidazol) verwendet werden. Die Erfindung umfaßt jedoch auch die Verwendung ähnlicher Verbindungen, die entsprechend reagieren.

Es müßte also sehr überraschen, daß einige spezielle Verbindungen dennoch eine ungestörte Messung erlauben. Das muß besonders beim 5HYC-AA verwundern, denn die Grundreaktion zwischen 2,4-Dichlorphenol-6-sulfonsäure und und 4-Aminoantipyrin, die durch oxidative Kupplung den gleichen Farbstoff liefern, wird, wie alle PAPReaktionen, vollständig von Alaninsulfinsäure und Sulfit unterdrückt.

Weiterhin sind Gegenstand der Erfindung analytische Mittel zum Nachweis von GOT, die aus einer Mischung der für das obige Verfahren notwendigen Reagenzien bestehen. Ein im Sinne der Erfindung vorteilhaftes analytisches Mittel besteht aus einer Mischung der Reagenzien mit folgender Zusammensetzung:

a) 1 mmol α-Ketoglutarat
1 -20 mmol Alaninsulfinsäure
0,05- 10 mmol Chromogen
100 - 10.000 U Peroxidase
50 - 20.000 U Pyruvatoxidase
0,5 - 5 mmol lösliches Phosphat
1 - 100 mmol Puffer (pH 6-7)

10 - 1000 ml Wasser werden zur Herstellung der Reagenz- lösung zugesetzt, die je nach Größe der Testansätze und Konzentration der Untersuchungslösungen für ca. 100 - 1000 Teste verwendet werden kann.

Zur Aktivierung der Enzyme enthält die Reagenzmischung bevorzugterweise noch

b) 0,5 - 5 mmol lösliches Magnesiumsalz
0,05 - 1 mmol Thiaminpyrophosphat
0,01 - 0,1 mmol Flavinadenindinucleotid (FAD)

Die Reagenzien können in gebrauchsfertiger Mischung vorliegen, auf Teststreifen imprägniert sein, oder auch in aufeinander abgestimmten Mengen auf verschiedene zu einer Handelspackung vereinigte Behälter verteilt sein oder in einer oder mehreren Tabletten vorliegen, die gegebenenfalls mehrere Schichten mit unterschiedlicher Zusammensetzung aufweisen und erst zu der Reaktion durch Vermischen mit geeigneten Lösungsmitteln - beispielsweise einer Pufferlösung oder der Testflüssigkeit - zur gebrauchsfertigen Reagenzienmischung vereinigt werden.

Anhand der folgenden Beispiele sei die Erfindung weiter erläutert.

**Beispiel 1**

**Messung der GOT im Photometer**

Zu 1 ml folgender Lösung:
40 mmol 4-(2-Hydroxyethyl)-1-piperazinethansulfonsäure (HEPES)/Kaliumhydroxid, pH 6,7
200 mmol Alaninsulfinsäure
0,5 mmolKaliumdihydrogenphosphat
10 mmol Magnesiumchlorid
0,5 mmol Thiaminpyrophosphat

0,01 mmol Flavinadenindinucleotid
0,5 mmol Ethylendiamintetraessigsäure
0,1 mmol N-(3'- Sulfonyl-4'-hydroxy-5'-chlorphenyl)-4-aminoantipyrin
12 mmol α-Ketoglutarat
1000 U Peroxidase
2500 U Pyruvatoxidase
ad 1000 ml Wasser

werden 0,02 ml Humanserum gegeben. Meßbeginn nach 2 Minuten, T = 25°C, λ= 546 nm, ε = 19,6 $cm^2$λmol. Meßgröße ist das ΔE/min. bis 5 Minuten nach Meßbeginn. Die Messung kann zwischen 450 und 580 nm durchgeführt werden, jedoch muß für die gewünschten Wellenlängen das ε jeweils bestimmt werden,

**Beispiel 2**

**GOT im Teststreifen**

Zur Herstellung von Teststreifen werden 2 Papiere imprägniert:

**A Enzympap ier**

0,5 mol/l Mrpholinoethansulfonsäure (MES)/Kaliumhydroxid, pH 6,5
200 mmol/l Alaninsulfinsäure
1 mmol/l Kaliumdihydrogenphosphat
10 mmol/l Magnesiumchlorid
0,5 mmol/l Thiaminpyrophosphat
0,01 mmol/l FAD
20.000 U/l POD
250.000 U/l Py-OD

Mit dieser Lösung wird ein saugfähiges Papier, Dicke 50m Flächengewicht 12e/$m^2$, Saugfähigkeit 50 g $H_2O/m^2$, imprägniert und 5 Minuten bei 50°C getrocknet.

Das Papier wird anschließend auf 1 cm Breite geschnitten.

**B Indikatorpapier**

10 mmol/l 4,5-Bis-[4-dime yl-amino-phenyl]-2-(3,5-dimethoxy-4-hydroxyphenyl)-imidazol B-DDH-Imidazol
18 mmol/l c-KG
0,1 mmol/l HCl als Lösemittel

Mit dieser Lösung wird ebenfalls ein entsprechendes saugfähiges Papier imprägniert, 5 Minuten bei 50°C getrocknet und auf 1 cm Breite geschnitten. Das Material wird in besonders vorteilhafter Weise im Teststreifen entsprechend der Figur 1 verarbeitet. Dabei wird eine 1 cm breite, durchsichtige Polycarbonat-Folie, loo m dick, gemeinsam mit dem Indikatorpapier und dem Enzympapier, entsprechend Figur 1, einseitig auf einem Plastikstreifen befestigt, so daß die Folie nach außen, das Indikatorpapier in die Mitte, das Enzympapier nach innen kommt. Daneben wird ein 15 mm breites Glasfaservlies, Dicke 1,5 mm, Faserdicke ca. 2 µm aufgebracht, so daß das freie Ende der Folie und der getränkten Papiere noch 6 mm über das Vlies reichen. Dann wird in 6 mm breite Teststreifen geschnitten. Bringt man nun 15 µl Vollblut auf den Probenauftragsbezirk 2a), entsprechend Figur.1, so durchdringt innerhalb 30 bis 60 Sekunden der Plasmaanteil das gesamte Glasfaservlies auch unterhalb der durchsichtigen Folie, während die Erythrozyten im Bereich 2a) festgehalten werden. Durch Andrücken der Folie kommen nun das Enzymund das Indikatorpapier (4) mit dem entwickelten Plasma in Berührung und werden gleichmäßig durchfeuchtet. Die im Plasma enthaltene GOT reagiert unter Blaufärbung, deren Intensität proportional zur GOT-Menge ist und gegebenenfalls in einem Remissionsphotometer gemessen werden kann. In gleicher Weise reagieren auch die anderen Probematerialien wie Serum, Plasma etc..

Figur 1

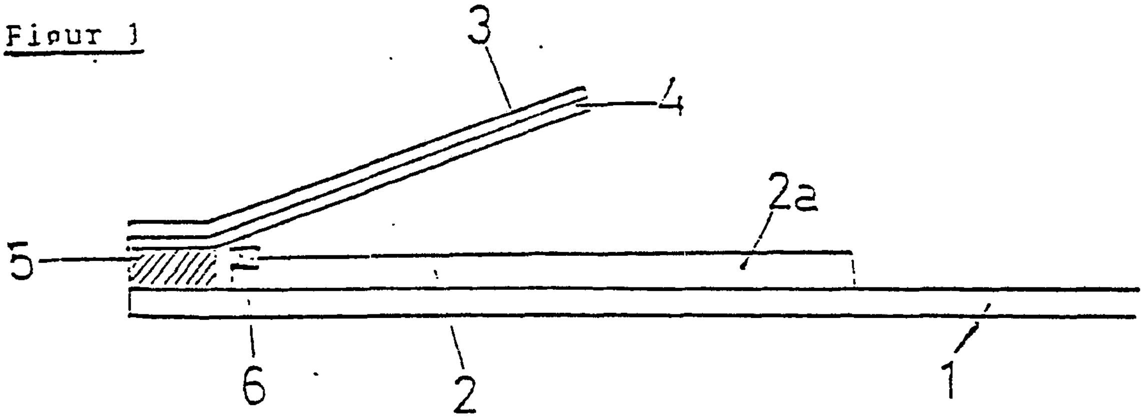


1 = Träger 4 = Enzym- und Indikatorpapier 2 = Glasfaservlies 5 = Befestigung 2a= Probenauftragsbezirk 6 = hydrophbierte Zone 3 = durchsichtige Folie

**Patentansprüche**

1. Verfahren zur Aktivitätsbestimmung von Glutamat- Oxalacetat-Transaminase (GOT) in wässrigen Lösungen, wobei

a) α-Ketoglutarat mit Alaninsulfinsäure unter Katalyse von GOT zu Pyruvat, Schwefeldioxid und Glutamat umgesetzt wird,

b) das entstandene Pyruvat mit Phosphat und Sauerstoff unter Katalyse von Pyruvatoxidase zu Acetylphosphat, Kohlendioxid und Wasserstoffperoxid reagiert,

c) das Wasserstoffperoxid und ein Chromogen unter Kata- lyse einer Peroxidase zu einem Farbstoff und Wasser umgesetzt wird und

d) die Menge des gebildeten Farbstoffes bestimmt wird, dadurch gekennzeichnet, daß als Chromogen N-(3'S ulfonyl-4'-hydroxy-5'-chlorphenyl)-4-aminoantipyrin (SHYC-AA) und 4,5-Bis-[4-dimethyl-aminophenyl]-2[3,5-dimethoxy-4-hydroxyphenyl]-imidazol (B-DDH-Imidazol) oder ein verwandter stoff verwendet wird.

2. Verfahren nach Anspruch 1, dadurch gekennzeichnet, daß die Reaktionen in Gegenwart eines wasserlöslichen Magnesiumsalzes, Thiaminpyrophosphat, Flavinadenindinucleotid, und/oder eines Puffers von pH 6-7 durchgeführt wird.

3. Verfahren nach Anspruch 1 oder 2, dadurch gekenn- zeichnet, daß die Reaktion in einer Küvette durchgeführt wird und die Menge des gebildeten Farbstoffs durch Messung der Lichttransmission gemessen wird.

4. Verfahren nach Anspruch 3, dadurch gekennzeichnet, daß die Reaktionslösung durch Auflösen eines Reagenzlyophilisats, einer Reagenztablette oder durch Ablösen der Reagenzien von einem damit imprägnierten Träger mittels der benötigten Menge Wasser, einer Pufferlösung oder der Testflüssigkeit hergestellt wird.

5. Verfahren nach Anspruch 1 oder 2, dadurch gekennzeichnet, daß die Reaktion auf einem mit den Reagenzien imprägnierten saugfähigen Träger durch Aufbringen der Testflüssigkeit stattfindet und der gebildete Farbstoff in Remission bestimmt wird.

6. Analytisches Mittel zur Aktivitätsbestimmung von GOT bestehend aus α-Ketoglutarat, Alaninsulfinsäure, Chromogen, Peroxidase, Pyruvatoxidase, löslichem Phosphat, Puffer und Wasser, dadurch gekennzeichnet, daß als Chromogen N-(3'-Sulfonyl-4'-hydroxy-5'-chlorphenyl) -4-aminoantipyrin (SHYC-AA)oder 4,5-Bis-[4-dimethylaminophenyl]-2- [3,5-dimethoxy-4-hydroxyphenyl]-imidazol (B-DDH-Imidazol) oder ein verwandter Stoff enthalten ist.

7. Analytisches Mittel gemäß Anspruch 6, dadurch ge- kennzeichnet, daß zusätzlich lösliches Magnesiumsalz, Thiaminpyrophosphat und Flavinadenindinucleotid (FAD) enthalten ist.

8. Analytisches Mittel gemäß Anspruch 6, bestehend aus 1 mmolα-Ketoglutarat, 1 -20 mmol Alaninsulfinsäure, 0,05 -lo mmol Chromogen, 100 - 10.000 U Peroxidase, 50.0 - 20.000 U Pyruvatoxidase, 0,5 - 5 mmol lösliches Phosphat, 1 - loo mmol Puffer (pH 6-7) und 10 - 1000 ml Wasser dadurch gekennzeichnet, daß als Chromogen N-(3'-Sulfonyl-4'-hydroxy-5'-chlorphenyl)4-aminoantipyrin (SHYC-AA) und 4,5-Bis [4-dimethylaminophenyl]-2-[3,5-dimethoxy-4-hydroxyphenyl]-imidazol (B-DDH-Imidazol) oder ein verwandter Stoff enthalten ist.

9. Analytisches Mittel gemäß Anspruch 8, dadurch gekennzeichnet,daß zusätzlich 0,5 - 5 mmol lösliches Magnesiumsalz, 0,05 - 1 mmol Thiaminpyrophosphat und/oder 0,01 - 0,1 mmol Flavinadenindinucleotid (FAD) enthalten ist.

10. Analytisches Mittel nach Anspruch 6, bestehend aus 40 mmol HEPES/KOH,(pH 6,7),200 mmol Alaninsulfinsäure, 0,5 mmol Kaliumdihydrogenphosphat, 10 mmol Magnesiumchlorid, 0,5 mmol Thiaminpyrophosphat, 0,01 mmol Flavinadenindinucleotid, 0,5 mmol Ethylendiamintetraessigsäure, 0,1 mmol N-(3'-Sulfonyl-4'-hydroxy-5'chlorphenyl)-4-aminoantipyrin 12 mmolα-Ketoglutarat, 1000 U Peroxidase, 2500 Pyruvatoxidase, 1000 ml Wasser.

11. Analytisches Mittel nach Anspruch 6, bestehend aus einem saugfähigen Träger, der mit Alaninsulfinsäure,

Peroxydase, Pyruvatoxidase, Phosphat und Puffersubstanzen imprägniert ist und einen zweiten saugfähigen Träger, der mit dem Chromogen und α-Ketoglutarat imprägniert ist und in saugfähigem Kontakt mit dem ersten Träger steht und einer Trägerschicht, auf der die beiden saugfähigen Schichten nebeneinander oder übereinander befestigt sind.

12. Analytisches Mittel, nach Anspruch 11, bestehend aus

a) einem Enzympapier welches mit einer Lösung aus 0,5 mmol/1 MES/KOH, pH 6,5, 200 mmol/1 Alaninsulfinsäure, 1 mmol/1 $KH_2PO_4$, 10 mmol/1 $MgCl_2$, 0,5 mmol/1 Thiaminpyrophosphat, 0,01 mmol/1 FAD, 20.000 U/1 POD und 250.000 U/1 Py-OD imprägniert ist und

b) einem Indikatorpapier, welches mit einer Lösung aus 10 mmol/1 B-DDH-Imidazol, 18 mmol/1α-KG und 0,1 mmol/1 HCl als Lösemittel imprägniert ist, die zusammen auf einer Trägerfolie befestigt sind.

**Revendications**

1. Procédé pour determiner l'activite de la transaminase glutamique- oxalacétique (GOT) dans des solutions aqueuses comportant les stades suivants:

a. on transforme de l'α-ketoglutarate avec de l'acide alanine-sulfi- nique sous l'effet catalytique du GOT en pyruvase, bioxyde de soufre et glutamate,

b. le pyruvate forme reagit avec du phosphate et de l'oxygène sous l'effet catalytique du pyruvate-oxydase pour donner du phosphate d'acètyle, du gaz carbonique et du peroxyde d'hydrogène.

c. Le peroxyde d'hydrogène et un chromogene sont transformes avec une peroxydase comme catalyseur en un colorant et de l'eau, et,

d. la quantité du colorant formé est dosée,

caractérisé en ce qu'on utilise comme chromogène la N-(3'-sulfonyl 4'-hydroxy 5'-chlorophényl)4-amino-antipyrine (SHYC-AA) et le 4,5-bis[4-diméthyl-aminophényl] 2-[3,5-diméthoxy 4-hydroxyphényl]imi dazole (BDDH-imidazole) ou une substance analogue.

2. Procede selon la revendication 1, caractérisé en ce que les réactions sont conduites en présence d'un sel de magnésium hydrosoluble, de pyrophosphate de thiamine, de dinucléotide de flavine-adenine, et/ou d'un tampon de pH 6 à 7.

3. Procede selon la revendication 1 ou 2, caractérisé en ce que la réaction est conduite dans une cuvette et la quantité de colorant formé est mesurée au moyen de la transmission de la lumière.

4. Procede selon la revendication 3, caractérisé en ce que la solution réactionnelle est préparée en dissolvant un lyophilisat de réactifs, un comprimé de réactifs ou en mettant en solution les réactifs à partir d'un support qui en est imprégné, à l'aide de la quantité d'eau nécessaire, d'une solution tamponnée ou du liquide à tester.

5. Procédé selon la revendication 1 ou 2, caractérisé en ce que la réaction a lieu sur un support absorbant imprégné des réactifs en mettant debout le liquide à tester et le colorant formé est dosé par rémission.

6. Agent analytique pour la détermination de l'activité du GOT composé d'α kétoglutarate, d'acide alanine-sulfinique, de chromogène, de peroxydase, de pyruvate-oxydase, de phosphate soluble, de tampon et d'eau, caractérisé en ce que le chromogene est la N-(3'-sulfonyl 4'-hydroxy 5'chlorophényl) 4-amino-antipyrine (SHYC-AA) ou le 4,5-bis-[4-diméthylaminoph ényl] 2- [3,5-diméthoxy 4-hydroxyphényl] imidazole (B-DDH-imidazole) ou une substance analogue.

7. Agent analytique selon la revendication 6, caractérisé en ce qu'il contient en outre un sel de magnésium soluble, du pyrophosphate de thiamine et du dinucléotide de flavine-adénine (FAD).

8. Agent analytique selon la revendication 6, composé de : 1 mmole d'α-kétoglutarate, 1 à 20 mmoles d'acide alaninesulfinique, 0,05 à 10 mmoles de chromogene, 100 à 10 000 U de peroxydase, 50 à 20 000 U de pyruvate-oxydase, 0,5 à 5 mmoles de phosphate soluble, 1 à 100 mmoles de tampon (pH 6 à 7) et de 10 à 1 000 ml d'eau, caractérisé en ce que le chromogène est la N-(3-sulfonyl 4'-hydroxy 5'-chlorophényl) 4-amino-antipyrine (SHYC-AA) et le 4,5-bis[4-diméthyl-aminophényl] 2-[3,5-diméthoxy 4-hydroxyphényl] imidazole (B-DDH-imidazole) ou une substance analogue.

9. Agent analytique selon la revendication 8, caractérisé en ce qu'il comporte en outre 0,5 à 5 mmoles de sel de magnésium soluble, 0,05 à 1 mmole de pyrophosphate de thiamine, et/ou 0,01 à 0,1 mmole de dinucléotide de flavine-adénine (FAD).

10. Agent analytique selon la revendication 6 composé de 40 mmoles de HEPES/KOH, (pH 6,7), 200 mmoles d'acide alanine-sulfinique, 0,5 mmole de dihydrogénophosphate de potassium, 10 mmole chloride de magnésie, 0,5 mmole ester pytophosphorique de thiamine, 0,01 mmole de dinucléotide de flavine-adénine, 0,5 mmole d'acide éthylènediaminetétracétique, 0,1 mmole de N-[3=sulfonyl 4'-hydroxy 5'-chlorophényl)4-amino-antipyrine, 12 mmoles d'α-kétoglutarate, 1 000 U de peroxydase, 2 500 U de pyruvate-oxydase et 1 000 ml d'eau.

11. Agent analytique selon la revendication 6 composé d'un support absor- bant imprégné d'acide alanine-sulfinique, de peroxydase, de pyruvateoxydase, de phosphate et de substances tampons et d'un second support absorbant imprégné de chromogene et d'α-kétoglutarate qui est en contact absorbant avec le premier support, et d'une couche de support sur laquelle les deux couches absorbantes sont fixées juxtaposées ou superposées.

12. Agent analytique selon la revendication 11, composé de :

a. un papier à enzymes imprégné d'une solution de 0,5 mmole/l de MES/KOH, pH 6,5, 200 mmoles/l d'acide alaninesulfinique, 1 mmole/l de $KH_2PO_4$, 10 mmoles/l de $MgCl_2$, 0,5 mmole /1 de pyrophosphate de thiamine,

0,01 mmole/l de FAD, 20 000 U/l de POD et 250 000 U/l de Py-OD et,

b. un papier indicateur imprégné d'une solution de 10 mmoles/l de B-DDH-imidazole, 18 mmoles/l d'α-KG et de 0,1 mmole/l de HCl comme solvant,

qui sont fixés ensemble sur une feuille de support.

**Claims**

1. Process for the determination of the activity of glutamate-oxalacetate-transaminabe (GOT) in aqueous solutions, whereby

a) -ketoglutarate is reacted with alanine-sulphinic acid, catalysed by GOT, to givw pyruvatw, sulphur dioxide and glutamate,

b) the resultant pyruvate is reacted with phosphate and oxygen, catalysed by pyruvate oxidase, to give acetyl phosphate, carbon dioxide and hydrogen peroxide,

c) the hydrogen peroxide and a chromogen is reacted. catalysed by a peroxidase, to give a coloured material and water and

d) the amount of coloured material formed is determined, characterised in that as chromogen there is used N-(3'sulphonyl-4'-hydroxy-5'-chlorophenyl)-4-aminoantipyrine (SHYC-AA) or 4,5-bis-[4-dimethylamjnophenyl)-2-(3,5dimethoxy-4-hydroxyphenyl]-imidazole (B-DDH-imidazole) or a related substance.

2. Process according to claim 1, characterised in that the reactions are carried out in the prehence of a water-soluble magnesium salt, thiamine pyrophosphate, flavime-adenine-dinucleotide and/or a buffer of pH 6-7.

3. Process according to claim 1 or 2, characterised in that the reaction is carried out in a cuvette and the amount of the coloured material formed is measured by light transmission.

4. Process according to claim 3, charactwrised in that the reagent solution is prepared by dissolving a reagent lyophilisate, a reagent tablet or by dissolving of the reagents from a carriwr impregnated therewith by means of the required amount of water, of a buffer solution or of the test liquid.

5. Procwss according to claim 1 or 2, characterised in that the reaction takes place on an absorbent carrier impregnated with the reagents by application of test liquid and the coloured material formed is determined by remission.

6. Analytical agent for the determination of the activity of GOT, consisting of -ketoglutarate, alanine-sulphimic acid, chromogen, peroxidase, pyruvate oxidase, soluble phosphate, buffer and water, characterised in that, as chromogwn, there is present N-(3'sulphonyl-4'-hydroxy-5'-chlorophenyl)-4-aminoantipyrine (SHYC-AA) or 4,5-bis-[4-dimwthylaminophenyl]-2-[3,5dimethoxy-4-hydroxyphenyl]-imidazole (B-DDH-imidazole) or a related substance.

7. Analytical agwnt according to claim 6, character- ised in that soluble magnesium salt, thiamine pyrophosphate and flavine-adenine-dinucleotide (FAD) is additionally present.

8. Analytical agent according to claim 6, consisting of 1 mmol -ketoglutarate, 1 - 20 mmol alanine-sulphinic acid, 0.05 - 10 mmol chromogen, 100 - 10,000 U peroxidase, 50 - 20,000 U pyruvate oxidase, 0.55 mmol soluble phosphate, 1 - 100 mmol buffer (pH 6 - 7) and 10 - 1000 ml. water, characterised in that, as chromogen, there is present N-(3'-sulphonyl-4'-hydroxy5'-chlorophenyl)-4-aminoantipyrine (SHYC-AA) or 4,5bis-[4-dimethylaminophenyl]-2-[3,5-dimethoxy-4-hydroxyphenyl]-imidazole (B-DDH-imidazole) or a related substance.

9. Analytical agent according to claim 8, character- ised in that 0.5 - 5 mmol of water-soluble magnesium salt, 0.05 - 1 mmol thiamine-pyrophosphate and/or 0.01 - 0.1 mmol flavine-adenine-dinucleotide (FAD) is additionally present.

10. Analytical agent according to claim 6, consisting of 4G mmol HEPES/ROH (pH 6.7), 200 mmol alaninesulphinic acid, 0.5 mmol potassium dihydrogen phosphate, 10 mmol magnesium chloride, 0.5 mmol thiamine pyrophosphate, 0.01 mmol flavine-adenine-dinucleotide, 0.5 mmol ethylenediamine-tetraacetic acid, 0.1 mmol N-(3'-sulphonyl-4'-hydroxy-5'-chlorophenyl)-4-aminoantipyrine, 12 mmol -ketoglutarate, 1000 U peroxidase, 2500 U pyruvate oxidase, 1000 ml. water.

11. Analytical agent according to claim 6, consisting of an absorbent carrier which is impregnated with alanine-sulphinic acid, peroxidase, pyruvate oxidase, phosphate and buffer substances and a second absorbent carrier which is impregnated with the chromogen and -ketoglutarate and is in absorbent contact with the first carrier and a carrier layer on which the two absorbent layers are fixed side-by-side or on top of one another.

12. Analytical agent according to claim 11, consist- ing of

a) an enzyme paper which is impregnated with a solution of 0.5 mmol/l. MES/ROH, pH 6.5, 200 mmol/l. alaninesulphinic acid, 1 mmol $RH_2PO_4$, 10 mmol/l. $MgCl_2$, 0.5 mmol/l. thiamine pyrophosphate, 0.01 mmol/l. FAD, 20,000 U/1. POD and 250,000 U/1. Py-OD and

b) an indicator paper which is impregnated with a solution of 10 mmol/l. B-DDH-imidazole, 18 mmol/l. -RG and 0.1 mmol/l. HCl as solvent,

which are fixed together on a carrier foil.